(19) Europäisches Patentamt
European Patent Office
Office européen des brevets

(11) **EP 3 349 006 A1**

(12) **EUROPEAN PATENT APPLICATION**
published in accordance with Art. 153(4) EPC

(43) Date of publication:
**18.07.2018 Bulletin 2018/29**

(51) Int Cl.:
**G01N 33/49** (2006.01)    **G01N 33/48** (2006.01)

(21) Application number: **16861864.3**

(22) Date of filing: **29.09.2016**

(86) International application number:
**PCT/JP2016/078940**

(87) International publication number:
**WO 2017/077796 (11.05.2017 Gazette 2017/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **06.11.2015 JP 2015218530**

(71) Applicant: **So, Shimbu**
**Tokyo 108-0075 (JP)**

(72) Inventor: **So, Shimbu**
**Tokyo 108-0075 (JP)**

(74) Representative: **Lavoix**
**Bayerstrasse 83**
**80335 München (DE)**

(54) **METHOD AND APPARATUS FOR TESTING IMMUNE STATE**

(57)    To provide a method capable of testing an immune state in more detail and in a simple and inexpensive manner to identify diseases such as cancer and to identify side-effects of treatment with an anticancer agent and the like, effects of immunotherapy, a state of prognosis, and the like. The method includes: a step of calculating the total number of white blood cells serving as immune cells in a body of a subject and the number of each of immune cell subgroups based on test information on blood counts of blood components and white blood cell images of the subject and analysis information on the CD classification of monoclonal antibodies that bind to surface antigens of white blood cells; and a step of analyzing the immune state of the subject based on a proportion and a change in the numbers of the immune cell subgroups.

FIG.2

Treg CELL — IMMUNOSUPPRESSION → CD$_4$ ↑ — Th2 CELL — B CELL ⟹ HUMORAL IMMUNITY ↑

NK CELL — INF−r → Th1 CELL    Th17 CELL    NKT CELL

CD$_4$ ⤢ GASTROINTESTINAL TRACT (INTESTINAL TRACT) AUTOIMMUNITY

CELLULAR IMMUNITY ↑ (killer−T=CD$_8$)

* ENHANCEMENT OF Th1 IMMUNITY = SYSTEMIC KILLING POWER OF CANCER
* SOLE KILLING POWER

- Th1 CELLULAR IMMUNITY = NK + CD$_8$ (KILLER T CELL)
- Th2 HUMORAL IMMUNITY = NKT + B CELL
- Th17 (GASTROINTESTINAL TRACT)
- Treg = IMMUNOSUPPRESSION (REGULATORY T−CELL)

**Description**

TECHNICAL FIELD

The present invention relates to a method and a testing apparatus an immune state. The test is carried based on a proportion of each of immune cell subgroups and the like.

BACKGROUND ART

Conventionally, an image inspection by means of CT, PET-CT, a gastroscope and the like, a blood test for detecting a substance which is contained in blood by using a tumor marker and the like have been carried out in order to diagnose cancer, to follow-up after treatment and the like. Among them, a blood test which uses a tumor marker has been often utilized in clinical settings because of its simplicity, and many effective tumor markers have been developed. In addition, a method of testing cancer which determines whether one is suffering from cancer or not based on an amount of a tumor marker per unit volume and the like are disclosed (for example, see PTL 1 to 3).

CITATION LIST

Patent Literature

Patent Literature 1: JP 2014-20930 A
Patent Literature 2: WO 00/58728 A
Patent Literature 3: JP 2015-141469 A

SUMMARY

Technical Problem

However, there are some cases where a value of a tumor marker increases even though there is no cancer expression, and there are other cases where a value of a tumor marker does not increase even though there is cancer. Therefore, it has been difficult to perceive a state of cancer only by a value of a tumor marker. In addition, since the method uses a special tumor marker the analysis costs high and the procedures are troublesome because of the use of a large-scale and expensive apparatus, a PCR technique and the like. As a result, it has been difficult to use the tumor marker in clinical settings. Further, it has been known that a therapeutic effect on cancer and the like vary depending not only on the presence of cancer cells but also on a patient's own immune strength. However, a simple and effective test which focuses on the immune strength has not yet been developed.

In view of the above problem, an object of the present invention is to provide a method of testing an immune state, which does not use an expensive testing apparatus or an expensive kit for testing, does not use a complex procedure such as a PCR technique, and is capable of testing an immune state of a subject in more detail in a simple and inexpensive manner.

Solution to Problem

In order to achieve the object described above, a method of testing an immune state according to the present application includes: a step of calculating a total number of white blood cells serving as immune cells in a body of a subject and a number of each of immune cell subgroups based on test information on blood counts of blood components and white blood cell images of the subject and analysis information on CD classification of monoclonal antibodies that bind to surface antigens of the white blood cells; and a step of analyzing the immune state of the subject based on a proportion and a change in the numbers of the immune cell subgroups.

Advantageous Effects

According to the present invention, it becomes possible to test an immune state of a subject in more detail and in a simple and inexpensive manner without requiring the use of an expensive testing apparatus or an expensive kit for testing and without requiring a complex procedure such as a PCR technique, based on test results of blood counts and white blood cell images obtained by a blood collection test generally performed in clinical settings as well as analysis results of the CD classification of monoclonal antibodies which bind to surface antigens which are present on the surfaces of white blood cells. Further, based on this immune state, it becomes possible to find out disease such as cancer and to perceive effects of medication, side effects of anticancer treatment and the like, effects of immunotherapy, a state of prognosis and the like in a simple and clear manner.

BRIEF DESCRIPTION OF DRAWINGS

[Fig. 1A] Fig. 1A shows an image of a list of test results (test information) of blood counts of blood components and white blood cell images which are used for a method of testing the immune state according to an embodiment of the present invention.

[Fig. 1B] Fig. 1B shows an image of a list of analysis results (analysis information) of CD numbers of surface antigens of white blood cells, which are used for the method of testing the immune state according to an embodiment of the present invention.

[Fig. 2] Fig. 2 is an explanatory view for explaining the correlation of cellular immunity and humoral immunity in immune cells.

[FIG. 3] Fig. 3 is an explanatory drawing for explaining test results of the method for testing the immune state according to the present embodiment, Fig. 3(A) shows a pie chart which shows the proportion of each of the components of white blood cell subgroups in a patient with renal cancer, and the total number (the number) of each of the components calculated according to the calculation formulae (1) to (12), Fig. 3(B) is a pie chart which shows the proportions of monocytes and subgroups of lymphocytes, and Fig. 3(C) is a pie chart which shows the proportions only of lymphocyte subgroups.

[FIG. 4A] Fig. 4A is an explanatory view for explaining an example of test results of the method of testing the immune state according to the present embodiment, and shows test results of a patient with end-stage esophageal cancer.

[FIG. 4B] Fig. 4B is an explanatory view for explaining an example of test results of the method of testing the immune state according to the present embodiment, and shows test results of a healthy subject.

[FIG. 4C] Fig. 4C is an explanatory drawing for explaining an example of test results of the method of testing the immune state according to the present embodiment, and shows test results of a patient with colon cancer who had been under the treatment with an anticancer agent until one year ago.

[FIG. 5] Fig. 5 is an explanatory drawing for explaining examples of test results of the method for testing the immune state according to the present embodiment, (A) shows test results of a patient with chronic rheumatoid arthritis, and (B) shows test results of a patient with atopic dermatitis.

[FIG. 6A] Fig. 6A is an explanatory view for explaining a structure chart of the hardware of the testing apparatus.

[Fig. 6B] Fig. 6B is an explanatory view for explaining a functional block chart of the testing apparatus.

DESCRIPTION OF EMBODIMENT

Hereinafter, an embodiment of the present invention is described. A method of testing an immune state according to the present embodiment has a step of calculating a total number of white blood cells serving as immune cells in a body of a subject and a number of each of immune cell subgroups based on test information on blood counts of blood components and white blood cell images of the subject and analysis information on the CD classification of monoclonal antibodies that bind to surface antigens of white blood cells, and a step of analyzing the immune state of the subject based on a proportion of and a change in the numbers of the immune cell subgroups.

The method of testing the immune state according to the present embodiment has the steps as described above, and makes it possible to test an immune state of a subject in a simple and inexpensive manner without requiring the use of an expensive testing apparatus or an expensive test kit as well as without requiring a complex procedure such as a PCR technique, by finding out states (proportions) of the immune cell subgroups in blood. In addition, a method of testing an immune state which makes it possible to provide test results which enable finding out various diseases such as cancer and diseases involved in immunity at an early stage and understanding effects of medication, side effects of anticancer treatment and the like, effects of immunotherapy, a state of prognosis and the like is provided.

Nowadays, it is difficult to remove cancer completely except for a few cancers in the space of humoral immunity (mainly blood cancer) despite chemotherapy using a potent anticancer agent. Effects of cancer treatment should not be determined only by using the change in the size of cancer as a determination criterion. The determination of effects of cancer treatment also requires consideration of the rest of life of the patient. It is thought that therapeutic effects of cancer and the rest of life vary depending on the patient's own physical strength (immune strength). A decrease in the immune strength (decrease in CD4-positive T cells) causes the metastasis and growth of cancer cells. Though the kind of new anticancer agents has increased, the cases where cancer metastasizes to another organ after the anticancer treatment are often observed. In many cases, cancer metastases occur at a cellular level of micrometastases (micrometastatic lesions) and are invisible to the naked eye. Accordingly, unless cancer cells are completely extinguished, the body of the patient falls into a state where the body has no ability against the metastasis of cancer cells, in a state where the immune strength decreases by the use of an anticancer agent. It is thought, as a result, that systemic metastases of the cancer may occur.

In addition, it is thought that one should also have doubts about using unilaterally a systemic radiation therapy and chemotherapy from the viewpoint of QOL (Quality Of Life). This is because it has been clear according to recent statistics that there is only a three-month difference in the rest of lives of cancer patients between the case where an anticancer agent is used and the case where no anticancer agent is used. It is thought that this is caused by the decrease in the immune strength of patients (decrease in CD4-positive T cells) due to the radiation therapy and the chemotherapy. Additionally, it has been verified, as a result of the inventor's validation of the previous statistics of clinical therapies, that the strength of autoimmunity affects the prognosis of cancer treatment (a prospect of the degree of recovery after illness, surgery and the like), that is, the survival rate.

For example, in patients with primary liver cancer after surgery, the strength of the activity of NK cells (natural killer cells) which are involved in natural immunity greatly affects the survival rate of the patients. Accordingly, it can be understood that the activity of NK cells plays an important role in relapse prevention of cancer. In addition, when comparing 7-year survival rate of a group of patients with lung cancer after surgery who received a cellular immunotherapy and that of a group of patients who received only a traditional remedy, there was a two-fold difference. Further, there was a significant difference between the survival rate of a group of patients with liver cancer who received both a cellular immunotherapy and an MIS treatment and that of a group of patients with liver cancer who received only an MIS treatment. From the analysis on the above-described statistical data, it has been found that the prognosis of a cancer treatment varies depending on autoimmune states. In other words, a better immune state provides a better prognosis.

Since a novel immunotherapeutic agent against metastatic malignant melanoma (melanoma) of Ono Pharmaceutical Co., Ltd. was disclosed in New England Journal of Medicine, the age emphasizing immunotherapy has started in the medical world after genetic analyses. Nivolmab, i.e., therapeutic agent of a PD1 antibody, developed by Ono Pharmaceutical Co., Ltd., acts on activated lymphocytes and plays a role in triggering further activation. Accordingly, using this type of the immunotherapeutic agents may cause severe side effects on patients with intractable autoimmune diseases other than cancer patients, such as patients with multiple sclerosis, rheumatism and systemic lupus erythematosus. Therefore, in order to find out a method of treatment which is capable of avoiding side effects to the most, development of a method of testing which makes it possible to elucidate a change in each of subgroups of immune cells in a simple and inexpensive manner by perceiving the immune state of a patient has been required.

In addition, physiological and pathological roles of each of subgroups of immune cells in our bodies have been gradually elucidated through the resent clinical researches. Elucidation of the roles of immune cells and the flow (the mechanism) of immunity is essential not only in the process of treatment of cancer, but also in the process of treatment of diseases related to immunity (such as connective tissue disease, chronic viral infection and acute viral infection). In order to elucidate the above, it is required to perceive the number and the ratio of each of the immune cell subgroups.

However, in the course of clinical therapies at present, methods capable of verifying the body's immune system are limited, and costs for such clinical tests are high. Therefore, elucidating immunity has not yet been going smoothly.

In view of the above, the inventor found out that a state of immune strength of a patient can be perceived by analyzing a proportion of each of immune cells subgroups (white blood cell subgroups) in white blood cells, and has completed the present invention. That is, the inventor has made it possible to test an immune state in a simple manner, based on blood counts (complete blood cell count) and white blood cell images (white blood cell demarcation) in a blood collection test which is generally performed in clinical settings, as well as analysis results of the CD classification (CD: cluster of differentiation) of monoclonal antibodies which bind to surface antigens which are present on the surfaces of white blood cells.

White blood cells are roughly divided into three kinds, i.e., granulocytes, monocytes (mononuclear cells) and lymphocytes. Granulocytes are further divided into three subgroups, i.e., eosinophils, neutrophils, and basophils. Monocytes are present as a subgroup of mononuclear leukocytes in blood.

In addition, the lymphocyte subgroup includes helper T cells (CD4-positive T cells), killer T cells (CD8-positive T cells), B cells, NK (natural killer) cells, and NKT (natural killer T) cells. The B cells and the NKT cells are involved in Th2 humoral immunity such as production of antibodies. The NK cells and the killer T cells are involved in Th1 cellular immunity such as removal of virus-infected cells, cancer cells and wasted cells. The helper T cells take charge of the center of immune system.

The granulocyte subgroups (eosinophils, basophils, and neutrophils), monocytes, and the NK cells are innate immune cells. The killer T cells, the helper T cells, the B cells and the like are adaptive immune (acquired immune) cells. The NKT cells have both the properties of innate immune cells and those of adaptive immune cells. Further, the NK cells and the NKT cells are cells of an early induction reaction, which determine the flow of immunity that is induced at several hours after infection.

Accordingly, it is possible to identify a state of the body's immune system (the health degree), a flow of the body's immune system, immune abnormality at early stage, and states from effects of medication to therapeutic effects (states from therapeutic effects to side effects) against cancer and each of other diseases, by analyzing states of the above-described immune cell subgroups.

Hereinafter the method of testing the immune state according to the present embodiment is described in detail.

First, test results (test information) of blood counts of blood components and white blood cell images of a subject are obtained. From the test information, the number of white blood cells and white blood cell images are obtained. As a method of testing blood counts and white blood cell images, a method of testing which is generally used in clinical settings may be used. For example, by collecting blood from a subject and requesting a clinical center or the like to conduct a test, the test information can be obtained without having an expensive testing apparatus and the like in a simple and inexpensive manner.

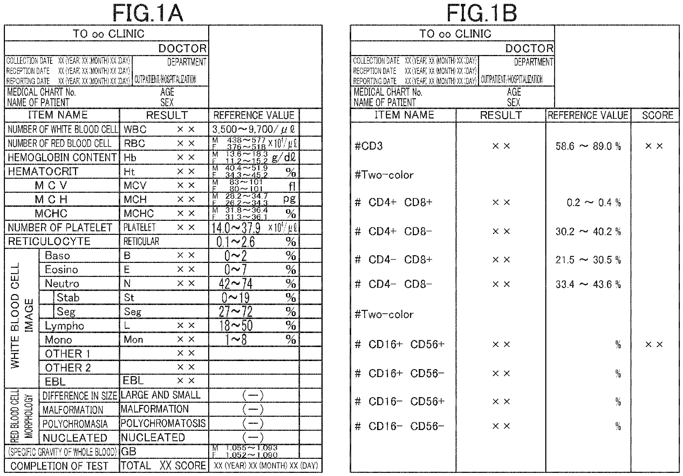

Fig. 1A shows an image of a list of test results of blood counts of blood components and white blood cell images of a subject. In Fig. 1A, "WBC" is the number of the white blood cells in 1 μl, and among the items of the white blood cell images (information on fractionation of white blood cells), "Baso" is a proportion (%) of basophils in the white blood cells, "Eosino" is a proportion (%) of eosinophils therein, "Neutro" is a proportion (%) of neutrophils therein, "Lympho" is a proportion (%) of lymphocytes therein, and "Mono" is a proportion (%) of monocytes therein.

Next, analysis results (analysis information) of CD numbers of surface antigens of the white blood cells (immune cells) are obtained. As an analysis method, a known analysis method may be used. For example, it is preferable that the analysis is performed by a Two Color Flow Cytometry analysis method. In this case, analysis results obtained in a clinical center or the like may be also used.

For the analysis of the CD classification, CD3, CD4, CD8, CD16 and CD56 are used. CD3 is an antigen which is present on membrane surfaces of all the T cells. CD4 is an antigen which is present on membrane surfaces of the helper T cells (CD4-positive T cells). CD8 is an antigen which is present on membrane surfaces of the killer T cells (CD8-positive T cells). CD16 is an antigen which is present on membrane surfaces of the NK cells. CD56 is an antigen which is present on membrane surfaces of the NK cells and the NKT cells.

Hereinafter, positive is also referred to as "+", and negative is also referred to as "-". The helper T cells express a property of "CD3+CD4+", the killer T cells express that of "CD3+CD8+", the B cells express that of "CD3-CD56-", the NK cells express that of "CD3-CD56+", and the NKT cells express that of "CD3+CD56+", respectively.

Fig. 1B shows an image of a list of analysis results (analysis information) of the CD classification according to a Two Color Flow Cytometry analysis method.

Next, the total number of the white blood cells (immune cells) and the total number (the number) of each of the white blood cell subgroups (immune cell subgroups) in the body of the subject are calculated based on the number of the white blood cells obtained from the test information ("WBC" in Fig. 1A) and the information on fractionation of the white blood cells ("Baso", "Eosino", "Neutro", "Lympho" and "Mono" in Fig. 1A), according to the following calculation formulae (1) to (5). In the case when the subject is male, the total number of the white blood cells is calculated according to the calculation formula (1). In the case when the subject is female, the total number of the white blood cells is calculated according to the calculation formula (2).

[Math. 1]

$$\text{The total number of white blood cells} = \text{the number of white blood cells } (/\mu l) \times 5,000 \text{ (blood volume) ml} \times 1,000 \text{ } (1,000 \text{ } \mu l = 1 \text{ ml}) \quad (1)$$

$$\text{The total number of white blood cells} = \text{the number of white blood cells } (/\mu l) \times 4,500 \text{ (blood volume) ml} \times 1,000 \text{ } (1,000 \text{ } \mu l = 1 \text{ ml}) \quad (2)$$

$$\text{The total number of granulocytes} = \text{the total number of white blood cells} \times \text{granulocytes (basophils + eosinophils + neutrophils) (\%)} \quad (3)$$

$$\text{The total number of monocytes} = \text{the total number of white blood cells} \times \text{monocytes (\%)} \quad (4)$$

$$\text{The total number of lymphocytes} = \text{the total number of white blood cells} \times \text{lymphocytes (\%)} \quad (5)$$

Next, the total number of the T cells, the total number of the B cells, the total number of the NK cells and the total number of the NKT cells in the lymphocyte subgroup are calculated based on the calculated total number of the lymphocytes and analysis information on the CD classification shown in Fig. 1B. With regard to the T cells, the total number of the helper T cells and the total number of the killer T cells are calculated.

First, the total number of the T cells is calculated according to the following calculation formula (6). The total number of the helper T cells and the total number of the killer T cells are calculated based on the calculated total number of the T cells according to the following calculation formulae (7) and (8).

Next, the total number of the non-T cells other than the T cells is calculated based on the total number of the T cells and the total number of the lymphocytes according to the following calculation formula (9). The total number of the B cells, the total number of the NK cells and the total number of the NKT cells are calculated based on the calculated total number of the non-T cells and the total number of the T cells according to the following calculation formulae (10), (11) and (12). Meanwhile, the number of each of the components in 1 μl can be calculated according to each of the above-described calculation formulae.

[Math. 2]

$$\text{The total number of T cells} = \text{the total number of lymphocytes} \times \text{CD3(+)} \ (\%) \quad (6)$$

$$\text{The total number of CD4(+) T cells} = \text{the total number of T cells} \times \text{CD4(+)} \ (\%) \quad (7)$$

$$\text{The total number of CD8(+) T cells} = \text{the total number of T cells} \times \text{CD8(+)} \ (\%) \quad (8)$$

$$\text{The total number of non-T cells} = \text{the total number of lymphocytes} \times (100 - \text{CD3(+)})) \ (9)$$

$$\text{The total number of B cells} = \text{the total number of non-T cells} \times (\text{CD16(-)/CD56(-)}) \ (\%) \quad (10)$$

$$\text{The total number of NK cells} = \text{the total number of non-T cells} \times ((\text{CD16(+)/CD56(+)}) + (\text{CD16(+)/CD56(-)})) \ (\%) \ (11)$$

$$\text{The total number of NKT cells} = \text{the total number of T cells} \times ((\text{CD16(-)/CD56(+)}) \ (\%) \quad (12)$$

An immune state of a subject can be analyzed in a simple manner based on an increase or decrease and a proportion of each of the granulocytes (eosinophils, basophils and neutrophils), the monocytes, and the lymphocytes (helper T cells, killer T cells, B cells, NK cells and NKT cells), calculated as described above. Meanwhile, the calculation which uses the above-described calculation formulae and the analysis of the immune state hereinafter can be performed in a faster and more accurate manner by performing the calculation and the analysis using a testing apparatus described below.

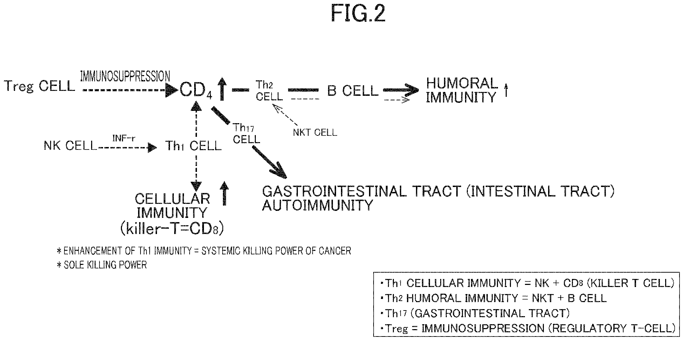

Fig. 2 roughly shows the correlation of cellular immunity and humoral immunity in immune cells. As shown in Fig. 2, the helper T cells (CD4-positive T cells) are divided into three kinds, i.e., Th1 cells, Th2 cells and Th17 cells. The Th1 cells mainly produce IFN-γ and are involved in activation of cellular immunity and the like. The Th2 cells mainly produce interleukin-4 (IL-4) and are involved in humoral immunity. The Th17 is present often in the gastrointestinal tract such as small intestine, produces interleukin-17 (IL-17), and is involved in autoimmunity such as inflammatory response. Besides them, regulatory T-cells (Treg) which play a role in inhibitory regulation against excessive immune response (immune tolerance) or the like are present as a kind of T cells.

A state of Th1 cellular immunity in which the Th1 cells are involved can be identified by analyzing the sum of the number of the NK cells and the number of the killer T cells, the increase or decrease in them and the like. In addition, a state of Th2 humoral immunity in which the Th2 cells are involved can be identified by analyzing the sum of the number of the NKT cells and the number of the B cells, the increase or decrease in them and the like.

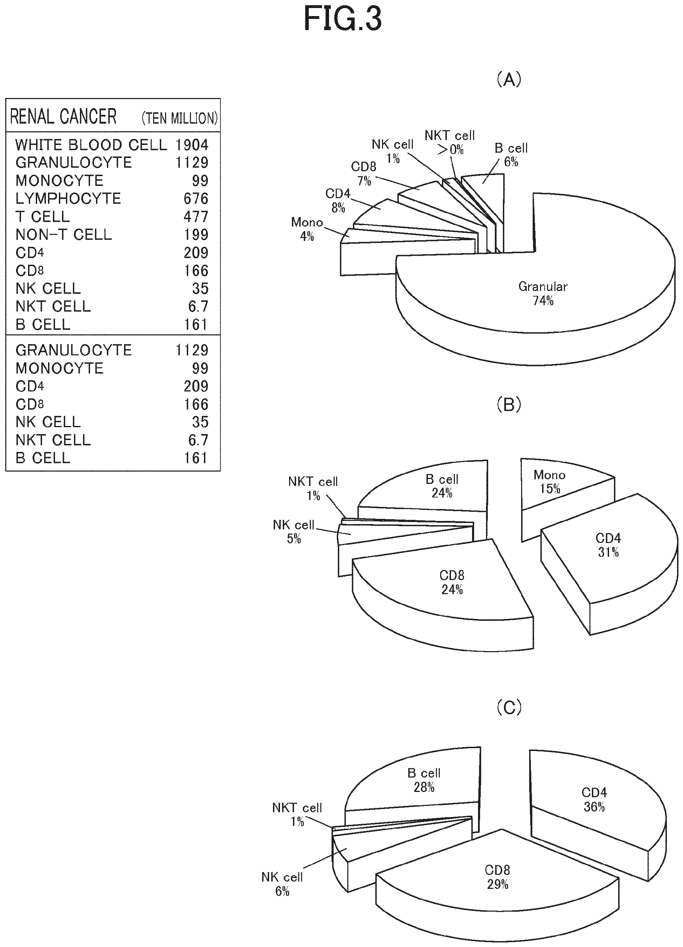

Examples of test results obtained by the method of testing the immune state according to the present embodiment are shown in Figs. 3A to 3C. Figs. 3A to 3C show test results of the immune state (the numbers of white blood cell subgroups) of a woman with renal cancer after surgery and pie charts of the proportions of white blood cell subgroups based on the test results. Fig. 3(A) shows a pie chart which shows the proportion of each component of white blood cell subgroups, and an example of the calculation of the numbers of white blood cell subgroups calculated based on the proportions on the left side of the pie chart. Fig. 3(B) is a pie chart which shows the proportion of the monocyte subgroup and the proportions of lymphocyte subgroups. Fig. 3(C) is a pie chart which only shows the proportions of lymphocyte subgroups.

From each of pie charts of Figs. 3A to 3C, it is understood that the proportions of lymphocytes in white blood cells are in a healthy state. In addition, Figs. 3A to 3C show a state of cellular immunity > humoral immunity and it is understood that Fig. 3 shows patterns of strong anticancer immunity. Further, the ratio of granulocytes and monocytes and lymphocytes shows a good balance, and shows a state of Th1 (NK cells + killer T cells) > Th2 (NKT cells + B cells). From the ratio, it is understood that the state is that of the cellular immunity dominance. In this way, it is understood that the postoperative progress is favorable.

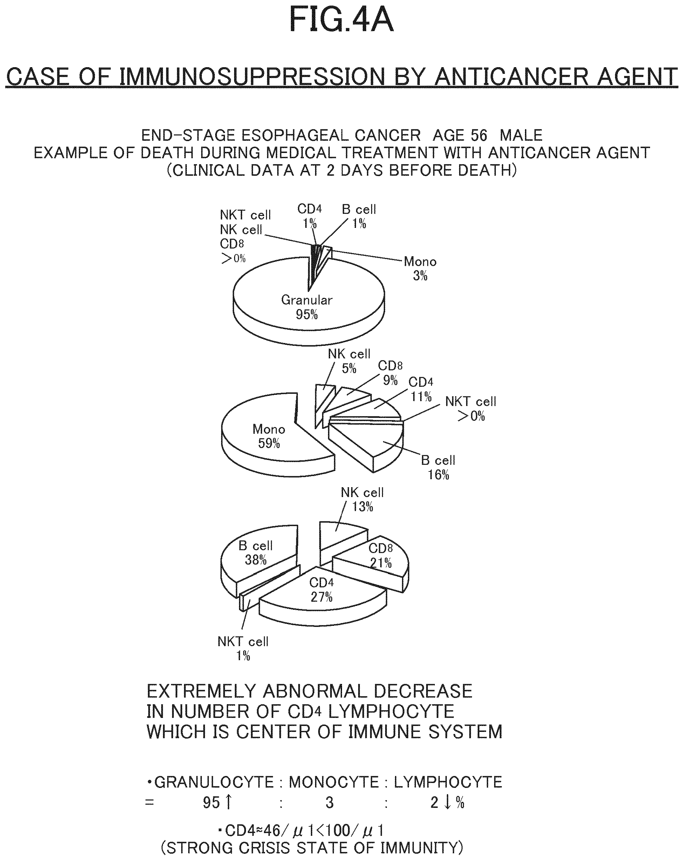

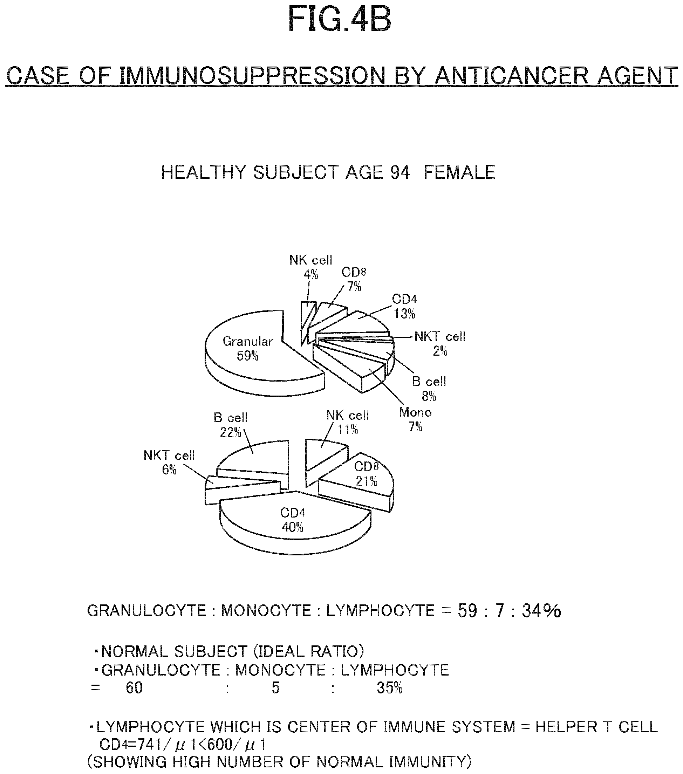

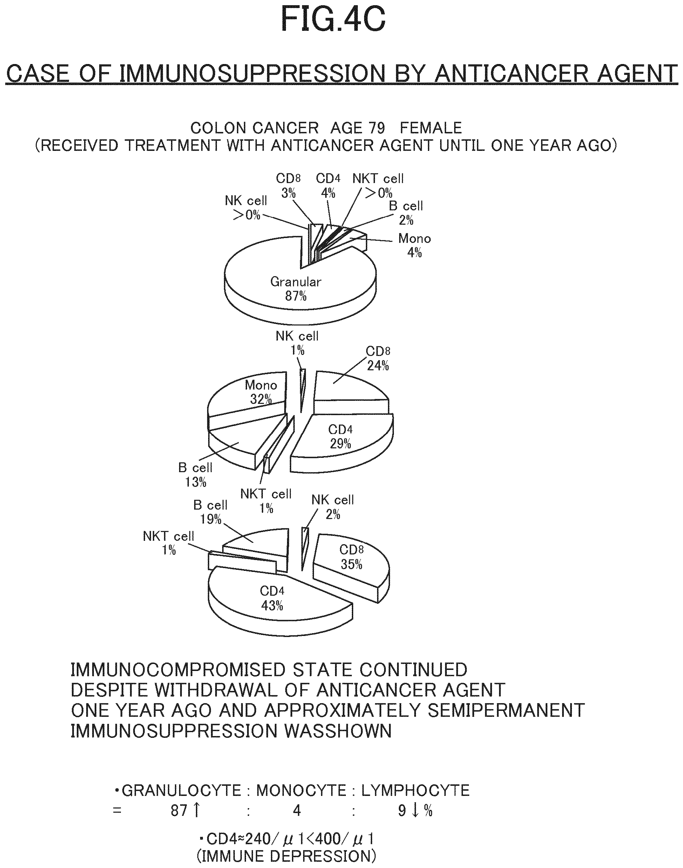

Next, the determination criteria of the analysis are described with analyzing immune states based on test results of immune cell subgroups (white blood cell subgroups) of three cases shown in Figs. 4A to. 4C. Fig. 4A shows test results of a patient (male) with end-stage esophageal cancer under medical treatment with an anticancer agent. Fig. 4B shows test results of a healthy subject (normal) who is an elderly (female). Fig. 4C shows test results of a patient (female) with colon cancer who had been under the treatment with an anticancer agent until one year ago. Each of the figures shows pie charts which show the proportions of the white blood cell subgroups, the total numbers of the white blood cell subgroups, and the like.

First, the health degree of the immune state can be identified by checking the balance between the sympathetic nerve and the parasympathetic nerve. Accordingly, the method of testing the immune state according to the present embodiment is suitable for medical checkup and screening. The ideal ratio of white blood cell subgroups is granulocytes (eosinophils, basophils and neutrophils) : monocytes : lymphocytes = 60 : 5 : 35 (%).

A state where the number of granulocytes increases (shown by ↑ in the figures) is the state of the sympathetic nerve dominance (due to infection, stress by the use of an anticancer agent, being at a late stage to an end stage of cancer and the like). In addition, a state where the number of lymphocytes increases (↑) is the state of the parasympathetic nerve dominance (due to being with autoimmune disease, allergic disease, hypersensitivity, obesity and the like).

The case shown in Fig. 4A is a patient with end-stage cancer who is under medical treatment with an anticancer agent, and shows an abnormal decrease (↓) in lymphocytes. The case shown in Fig. 4B is a healthy elderly, and shows an ideal ratio of granulocytes and lymphocytes, and a good balance between the sympathetic nerve and the parasympathetic nerve. The case shown in Fig. 4C is a patient with colon cancer who had been under the treatment with an anticancer agent until one year ago. In the case shown in Fig. 4C, a persistent decrease in lymphocytes is observed even though one year has lapsed after the termination of medical treatment with an anticancer agent, and it is understood that the case is in a situation of the immunosuppression and the sympathetic nerve dominance.

In addition, the strength of autoimmunity is analyzed from the number of the subgroup of helper T cells (CD4) which are lymphocytes that play a role as the center of immune system. As a result, it is understood that the case shown in Fig. 4C is in an immunocompromised state and that the case shown in Fig. 4A is in a crisis state of immunity. From the test results of the immune states of the cases shown in Figs. 4A to 4C, the case where the number of CD4 > 600/$\mu$l is determined as healthy. The case where the number of CD4 > 400/$\mu$l is determined as normal. The case where the number of CD4 < 400/$\mu$l is determined as being in an immunocompromised state. The case where the number of CD4 < 200/$\mu$l is determined as being in a crisis state of immunity. Accordingly, the method of testing the immune state according to the present embodiment is suitable for analyzing cancer treatment and analyzing cases of immunosuppression.

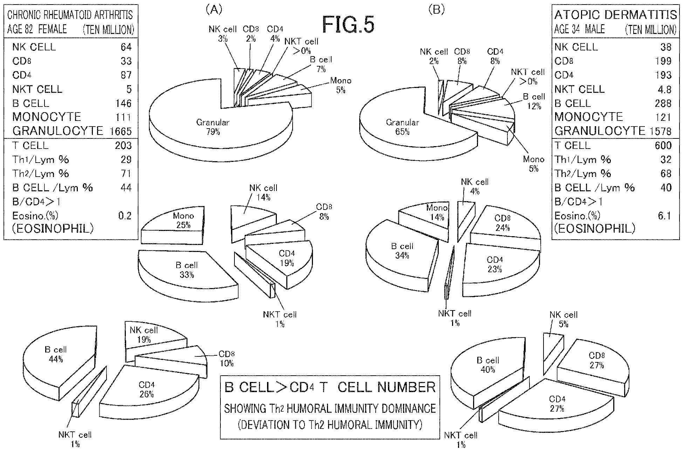

Next, immune states are analyzed based on test results of immune cell subgroups (white blood cell subgroups) of two cases other than cancer shown in Fig. 5. Fig. 5(A) shows test results obtained from a patient with chronic rheumatoid arthritis, and Fig. 5(B) shows test results obtained from a patient with atopic dermatitis. By analyzing these test results, the flow of the body's immune system and the habitus of immunity become apparent. Accordingly, the method of testing the immune state according to the present embodiment is suitable for analyzing the body's immune system such as connective tissue disease and allergic disease.

In the cases shown in Figs. 5A and 5B, states of B cells/helper T cells (CD4) > 1, that is, states of B cells > helper T cells (CD4) are shown. The states show the Th2 humoral immunity dominance (the deviation to Th2 humoral immunity).

The body's immune system is analyzed based on the correlation of cellular immunity and humoral immunity shown in Fig. 2. In the case of Th2 immune abnormality (the deviation to Th2 humoral immunity and the like), allergic disease (atopic dermatitis), myasthenia gravis, chronic active hepatitis (HBsAg(-)), membranous glomerulonephritis, pemphigus vulgaris, hyperthyroidism or hypothyroidism, sarcoidosis and the like are considered. In addition, chronic rheumatoid arthritis, SLE, urticaria, bronchial asthma and the like are considered. Further, avian influenza, influenza, SARS and the like are considered.

In the case of Th1 immune abnormality (the deviation to Th1 cellular immunity and the like), contact dermatitis, an early stage of cancer, tuberculosis, fungal infection, viral hepatitis, plaque psoriasis, multiple sclerosis and the like are considered. In the case of Th17 immune abnormality, ulcerative colitis, Crohn's disease, allergic disease such as irritable bowel disease and the like are considered.

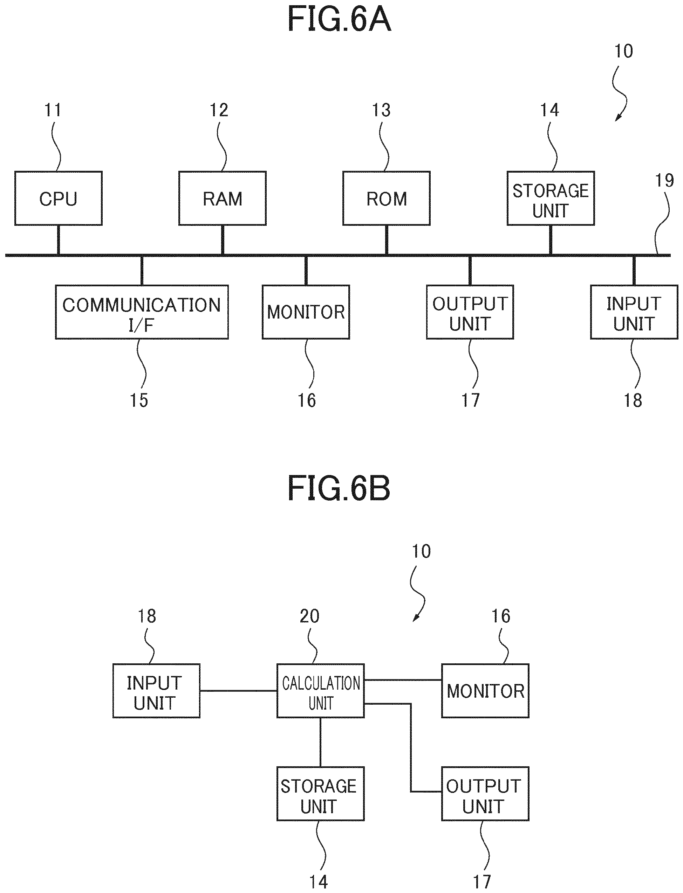

Next, a testing apparatus and a program suitable for executing the above-described method of testing are described. The testing apparatus can be constructed of an arithmetic unit having an arithmetic function such as a personal computer (PC), a tablet terminal, a PDA, a smartphone and a computing machine. An embodiment of the testing apparatus using a PC is described hereinafter with reference to Figs. 6A and Fig. 6B.

As shown in a structure chart of hardware shown in Fig. 6A, a testing apparatus 10 according to the present embodiment is mainly equipped with a CPU (Central Processing Unit) 11 which controls the whole operation of the testing apparatus 10, a RAM (Random Access Memory) 12 used as a work area of the CPU 11, a ROM (Read Only Memory) 13 for storing each of various kinds of programs such as a testing program executed by the CPU 11, a storage unit (memory) 14 composed of a recording medium such as a nonvolatile semiconductor memory (e.g., a flash memory), an SD card and an external hard disk, a communication I/F 15 connected to a communication network wiredly or wirelessly, a monitor (display unit) 16, an output unit 17 and an input unit 18. These constituent elements are electrically connected to each other by a bus 19. Besides them, the testing apparatus 10 has a component connected to the PC such as a CD-ROM drive and an external device I/F.

Next, functions of the testing apparatus 10 are described with reference to a functional block chart shown in Fig. 6B. As shown in Fig. 6B, the testing apparatus 10 includes the input unit 18, the storage unit 14, the monitor 16, the output unit 17, and the calculation unit 20.

The input unit 18 has a function of inputting test results of blood and analysis results of the CD classification into the calculation unit 20. As the input unit 18, for example, a keyboard, a mouse, and a touch panel may be used. A medical doctor and the like can directly input test results of blood and analysis results of the CD classification through the input unit 18. In addition, by an operation of the input unit 18, the information can be also sent to the calculation unit 20, by downloading through the internet communication and by copying from a USB memory and the like.

The storage unit 14 has a function of storing each kind of information. In the storage unit 14, analysis information on the CD classification of monoclonal antibodies that bind to surface antigens of white blood cells, which is described in the above-described explanation of the method of testing and the like are stored. The monitor 16 (display unit) has a function of displaying each kind of information such as a calculation results calculated by the calculation unit 20 and an analysis result. As the monitor 16, for example, a liquid crystal display may be used. The output unit 17 has a function of outputting each kind of information such as the calculation results calculated by the calculation unit 20 and analysis results. As the output unit 17, for example, a printer may be used.

The calculation unit 20 has a function of calculating the total number of white blood cells and the number of each of immune cell subgroups based on the analysis information obtained from the storage unit 14 and blood counts and test information inputted from the input unit 18, by executing the calculation formulae as describe above, a function of analyzing an immune state of a subject based on a proportion of and a change in the numbers of the immune cell subgroups, and a function of outputting an analysis result.

The program installed in the above-described testing apparatus 10 is a program for making a computer execute a step of calculating the total number of white blood cells serving as immune cells in the body of a subject and the number of each of immune cell subgroups based on test information on blood counts of blood components and white blood cell images of the subject and analysis information on the CD classification of monoclonal antibodies that bind to surface antigens of white blood cells in accordance with the calculation formulae as described above, and a step of analyzing the immune state of the subject based on a proportion and a change in the numbers of the immune cell subgroups. In the present specification, a program includes not only a program created by a computer programming language and installed in a PC, but also a function created by Excel or other software, a macro and the like.

By using the testing apparatus 10 having the above-described configuration, the total number of each of fractions of white blood cells can be calculated in a fast and accurate manner by the calculation unit 20. A medical doctor and a patient (a subject) can recognize the immune state visually and easily by displaying the calculation results on the monitor 16 or printing out the calculation results by a printer of the output unit 17. In addition, as shown in Figs. 3 to 5B, by displaying or printing out the calculation results and the analysis results by showing these results graphically or the like by the calculation unit 20, the change in the immune state and the like can be easily perceived. The calculation unit 20 makes it possible to show the results graphically and to edit a display screen and a print layout in a more accurate and faster manner.

Accordingly, the testing apparatus 10 and the program according to the present embodiment make it possible to test (analyze) the immune state of the subject in a simple and inexpensive manner. The testing apparatus 10 and the program according to the present embodiment is therefore suitably used for medical checkup and screening, analyzing cases of cancer treatment and immunosuppression, analyzing the body's immune system such as connective tissue disease and allergic disease and the like.

As described above, the method of testing and the testing apparatus 10 of the immune state according to the present embodiment make it possible to carry out analyses of cancer, diseases related to immunity and the like in a simple and inexpensive manner. As a result, it becomes possible to find out various kinds of cancer, diseases related to immunity and the like at an early stage, and to know effects of medication, side effects of anticancer treatment and the like, effects of immunotherapy, a state of prognosis and the like, in an easy and inexpensive manner.

As above, the embodiments of the present invention are described in detail by referring to the drawings, but specific constitutions are not limited to the embodiments or the examples, and the present invention encompasses modification of design to a degree that does not departs from the gist of the present invention.

CROSS-REFERENCE TO RELATED APPLICATION

The present application claims the right of priority based on Japanese Patent Application No. 2015-218530 filed on November 6, 2015 before the Japanese Patent Office, of which the whole disclosure is completely incorporated by reference in the present specification.

**Claims**

1. A method of testing an immune state, the method comprising:

   a step of calculating a total number of white blood cells serving as immune cells in a body of a subject and a number of each of immune cell subgroups based on test information on blood counts of blood components and white blood cell images of the subject and analysis information on CD classification of monoclonal antibodies that bind to surface antigens of the white blood cells; and
   a step of analyzing the immune state of the subject based on a proportion and a change in the numbers of the immune cell subgroups.

2. The method according to claim 1, wherein
   in the step of calculating the total number of the white blood cells and the number of each of immune cell subgroups,

   a number of granulocytes, a number of monocytes, and a number of lymphocytes in the immune cell subgroups are calculated based on the total number of the white blood cells and the analysis information on the CD classification;
   a number of T cells , a number of B cells, a number of NK cells, and a number of NKT cells in the lymphocytes are calculated based on the number of the lymphocytes and the analysis information on the CD classification; and

   in the step of analyzing the immune state,

   a proportion of each of the components is calculated based on the total number of the white blood cells, the number of the granulocytes, the number of the monocytes, the number of the lymphocytes, the number of the T cells, the number of the B cells, the number of the NK cells and the number of the NKT cells, and
   the immune state of the subject is analyzed based on the proportion and the change in the number,.

3. The method of testing an immune state according to claim 1 or 2, wherein
   the total number of the white blood cells is calculated according to following formula (1) or (2),
   a total number of the granulocytes, a total number of the monocytes and a total number of the lymphocytes are calculated according to following formulae (3), (4) and (5), respectively,
   a total number of the T cells is calculated according to following formula (6),
   a total number of the helper T cells and a total number of the killer T cells in the T cells are calculated based on the total number of the T cells according to following formulae (7) and (8),
   a total number of non-T cells other than the T cells is calculated according to following formula (9), and
   a total number of the B cells, a total number of the NK cells and a total number of the NKT cells are calculated based on the total number of the non-T cells and the total number of the T cells according to following formulae (10), (11) and (12):
   [Math. 1]

The total number of white blood cells = the number of white blood cells (/ μl) × 5,000 (blood volume) ml × 1,000 (1,000 μl = 1 ml) (1)

The total number of white blood cells = the number of white blood cells (/ μl) × 4,500 (blood volume) ml × 1,000 (1,000 μl = 1 ml) (2)

The total number of granulocytes = the total number of white blood cells $\times$ granulocytes (basophils + eosinophils + neutrophils) (%)　(3)

The total number of monocytes = the total number of white blood cells $\times$ monocytes (%)　(4)

The total number of lymphocytes = the total number of white blood cells $\times$ lymphocytes (%)　(5)

[Math. 2]

The total number of T cells = the total number of lymphocytes $\times$ CD3(+) (%)　(6)

The total number of CD4(+) T cells = the total number of T cells $\times$ CD4(+) (%)　(7)

The total number of CD8(+) T cells = the total number of T cells $\times$ CD8(+) (%)　(8)

The total number of non-T cells = the total number of lymphocytes $\times$ (100 − CD3(+))) (9)

The total number of B cells = the total number of non-T cells $\times$ (CD16(−)/CD56(−)) (%)　(10)

The total number of NK cells = the total number of non-T cells $\times$ ((CD16(+)/CD56(+)) + (CD16(+)/CD56(−))) (%)(11)

The total number of NKT cells = the total number of T cells $\times$ ((CD16(−)/CD56(+)) (%)　(12)

4. An testing apparatus of executing the method of testing an immune state according to any one of claims 1 to 3, the apparatus comprising:

a storage unit configured to store analysis information on CD classification of monoclonal antibodies that bind to surface antigens of white blood cells; and
a calculation unit configured to calculate a total number of the white blood cells serving as immune cells in a body of a subject and a number of each of immune cell subgroups based on the analysis information as well as test information on blood counts of blood components and white blood cell images of the subject, inputted from an input unit, and is configured to output an analysis result by analyzing the immune state of the subject based on a proportion and a change in the numbers of the immune cell subgroups.

## FIG.1A

| TO ○○ CLINIC | | | |
|---|---|---|---|
| | | DOCTOR | |
| COLLECTION DATE  XX (YEAR) XX (MONTH) XX (DAY)  RECEPTION DATE    XX (YEAR) XX (MONTH) XX (DAY)  REPORTING DATE    XX (YEAR) XX (MONTH) XX (DAY) | | DEPARTMENT  OUTPATIENT/HOSPITALIZATION | |
| MEDICAL CHART No.  NAME OF PATIENT | | AGE  SEX | |
| ITEM NAME | | RESULT | REFERENCE VALUE |
| NUMBER OF WHITE BLOOD CELL | WBC | × × | 3,500～9,700/μℓ |
| NUMBER OF RED BLOOD CELL | RBC | × × | M 438～577 ×10⁴/μℓ  F 376～518 |
| HEMOGLOBIN CONTENT | Hb | × × | M 13.6～18.3 g/dℓ  F 11.2～15.2 |
| HEMATOCRIT | Ht | × × | M 40.4～51.9 %  F 34.3～45.2 |
| M C V | MCV | × × | M 83～101 fl  F 80～101 |
| M C H | MCH | × × | M 28.2～34.7 pg  F 26.2～34.3 |
| MCHC | MCHC | × × | M 31.8～36.4 %  F 31.3～36.1 |
| NUMBER OF PLATELET | PLATELET | × × | 14.0～37.9 ×10⁴/μℓ |
| RETICULOCYTE | RETICULAR | | 0.1～2.6 % |
| WHITE BLOOD CELL IMAGE | Baso | B | × × | 0～2 % |
| | Eosino | E | × × | 0～7 % |
| | Neutro | N | × × | 42～74 % |
| | Stab | St | | 0～19 % |
| | Seg | Seg | | 27～72 % |
| | Lympho | L | × × | 18～50 % |
| | Mono | Mon | × × | 1～8 % |
| | OTHER 1 | | × × | |
| | OTHER 2 | | × × | |
| | EBL | EBL | × × | |
| RED BLOOD CELL MORPHOLOGY | DIFFERENCE IN SIZE | LARGE AND SMALL | (－) |
| | MALFORMATION | MALFORMATION | (－) |
| | POLYCHROMASIA | POLYCHROMATOSIS | (－) |
| | NUCLEATED | NUCLEATED | (－) |
| (SPECIFIC GRAVITY OF WHOLE BLOOD) | GB | | M 1.055～1.093  F 1.052～1.090 |
| COMPLETION OF TEST | TOTAL   XX SCORE | | XX (YEAR) XX (MONTH) XX (DAY) |

## FIG.1B

| TO ○○ CLINIC | | | |
|---|---|---|---|
| | | DOCTOR | |
| COLLECTION DATE  XX (YEAR) XX (MONTH) XX (DAY)  RECEPTION DATE    XX (YEAR) XX (MONTH) XX (DAY)  REPORTING DATE    XX (YEAR) XX (MONTH) XX (DAY) | | DEPARTMENT  OUTPATIENT/HOSPITALIZATION | |
| MEDICAL CHART No.  NAME OF PATIENT | | AGE  SEX | |
| ITEM NAME | RESULT | REFERENCE VALUE | SCORE |
| #CD3 | × × | 58.6 ～ 89.0 % | × × |
| #Two-color | | | |
| # CD4+  CD8+ | × × | 0.2 ～ 0.4 % | |
| # CD4+  CD8- | × × | 30.2 ～ 40.2 % | |
| # CD4-  CD8+ | × × | 21.5 ～ 30.5 % | |
| # CD4-  CD8- | × × | 33.4 ～ 43.6 % | |
| #Two-color | | | |
| # CD16+  CD56+ | × × | % | × × |
| # CD16+  CD56- | × × | % | |
| # CD16-  CD56+ | × × | % | |
| # CD16-  CD56- | × × | % | |

# FIG.2

Treg CELL ---IMMUNOSUPPRESSION---> CD$_4$ ↑ — Th2 CELL ===== B CELL ---> HUMORAL IMMUNITY ↑

NK CELL ---INF-r---> Th1 CELL

Th17 CELL      NKT CELL

CELLULAR IMMUNITY ↑ (killer-T=CD$_8$)

GASTROINTESTINAL TRACT (INTESTINAL TRACT) AUTOIMMUNITY

* ENHANCEMENT OF Th1 IMMUNITY = SYSTEMIC KILLING POWER OF CANCER
* SOLE KILLING POWER

- Th1 CELLULAR IMMUNITY = NK + CD$_8$ (KILLER T CELL)
- Th2 HUMORAL IMMUNITY = NKT + B CELL
- Th17 (GASTROINTESTINAL TRACT)
- Treg = IMMUNOSUPPRESSION (REGULATORY T-CELL)

# FIG.3

| RENAL CANCER | (TEN MILLION) |
|---|---|
| WHITE BLOOD CELL | 1904 |
| GRANULOCYTE | 1129 |
| MONOCYTE | 99 |
| LYMPHOCYTE | 676 |
| T CELL | 477 |
| NON-T CELL | 199 |
| CD4 | 209 |
| CD8 | 166 |
| NK CELL | 35 |
| NKT CELL | 6.7 |
| B CELL | 161 |
| GRANULOCYTE | 1129 |
| MONOCYTE | 99 |
| CD4 | 209 |
| CD8 | 166 |
| NK CELL | 35 |
| NKT CELL | 6.7 |
| B CELL | 161 |

(A)

(B)

(C)

# FIG.4A

## CASE OF IMMUNOSUPPRESSION BY ANTICANCER AGENT

END-STAGE ESOPHAGEAL CANCER  AGE 56  MALE
EXAMPLE OF DEATH DURING MEDICAL TREATMENT WITH ANTICANCER AGENT
(CLINICAL DATA AT 2 DAYS BEFORE DEATH)

NKT cell
NK cell
CD8
>0%

CD4
1%

B cell
1%

Mono
3%

Granular
95%

NK cell
5%

CD8
9%

CD4
11%

NKT cell
>0%

Mono
59%

B cell
16%

NK cell
13%

B cell
38%

CD8
21%

CD4
27%

NKT cell
1%

EXTREMELY ABNORMAL DECREASE
IN NUMBER OF CD4 LYMPHOCYTE
WHICH IS CENTER OF IMMUNE SYSTEM

·GRANULOCYTE : MONOCYTE : LYMPHOCYTE
=      95 ↑       :       3        :       2 ↓ %
·CD4≈46/ $\mu$ 1<100/ $\mu$ 1
(STRONG CRISIS STATE OF IMMUNITY)

EP 3 349 006 A1

# FIG.4B

## CASE OF IMMUNOSUPPRESSION BY ANTICANCER AGENT

HEALTHY SUBJECT AGE 94  FEMALE

GRANULOCYTE : MONOCYTE : LYMPHOCYTE = 59 : 7 : 34%

・NORMAL SUBJECT (IDEAL RATIO)
・GRANULOCYTE : MONOCYTE : LYMPHOCYTE
=    60        :    5     :      35%

・LYMPHOCYTE WHICH IS CENTER OF IMMUNE SYSTEM = HELPER T CELL
  CD4=741/ $\mu$ 1<600/ $\mu$ 1
(SHOWING HIGH NUMBER OF NORMAL IMMUNITY)

15

# FIG.4C

## CASE OF IMMUNOSUPPRESSION BY ANTICANCER AGENT

COLON CANCER   AGE 79   FEMALE
(RECEIVED TREATMENT WITH ANTICANCER AGENT UNTIL ONE YEAR AGO)

IMMUNOCOMPROMISED STATE CONTINUED
DESPITE WITHDRAWAL OF ANTICANCER AGENT
ONE YEAR AGO AND APPROXIMATELY SEMIPERMANENT
IMMUNOSUPPRESSION WASSHOWN

・GRANULOCYTE : MONOCYTE : LYMPHOCYTE
=      87 ↑      :      4      :      9 ↓ %

・CD4≈240/ $\mu$ 1<400/ $\mu$ 1
(IMMUNE DEPRESSION)

FIG.5

(A) / (B)

| CHRONIC RHEUMATOID ARTHRITIS AGE 82 FEMALE (TEN MILLION) | |
| --- | --- |
| NK CELL | 64 |
| CD8 | 33 |
| CD4 | 87 |
| NKT CELL | 5 |
| B CELL | 146 |
| MONOCYTE | 111 |
| GRANULOCYTE | 1665 |
| T CELL | 203 |
| Th1/Lym % | 29 |
| Th2/Lym % | 71 |
| B CELL /Lym % | 44 |
| B/CD4>1 | |
| Eosino.(%) (EOSINOPHIL) | 0.2 |

| ATOPIC DERMATITIS AGE 34 MALE (TEN MILLION) | |
| --- | --- |
| NK CELL | 38 |
| CD8 | 199 |
| CD4 | 193 |
| NKT CELL | 4.8 |
| B CELL | 288 |
| MONOCYTE | 121 |
| GRANULOCYTE | 1578 |
| T CELL | 600 |
| Th1/Lym % | 32 |
| Th2/Lym % | 68 |
| B CELL /Lym % | 40 |
| B/CD4>1 | |
| Eosino.(%) (EOSINOPHIL) | 6.1 |

B CELL>CD4 T CELL NUMBER
SHOWING Th2 HUMORAL IMMUNITY DOMINANCE
(DEVIATION TO Th2 HUMORAL IMMUNITY)

# FIG.6A

# FIG.6B

## INTERNATIONAL SEARCH REPORT

| International application No. |
|---|
| PCT/JP2016/078940 |

**A. CLASSIFICATION OF SUBJECT MATTER**
*G01N33/49(2006.01)i, G01N33/48(2006.01)i*

According to International Patent Classification (IPC) or to both national classification and IPC

**B. FIELDS SEARCHED**

Minimum documentation searched (classification system followed by classification symbols)
G01N33/49, G01N33/48

Documentation searched other than minimum documentation to the extent that such documents are included in the fields searched
Jitsuyo Shinan Koho         1922-1996   Jitsuyo Shinan Toroku Koho   1996-2016
Kokai Jitsuyo Shinan Koho   1971-2016   Toroku Jitsuyo Shinan Koho   1994-2016

Electronic data base consulted during the international search (name of data base and, where practicable, search terms used)
JSTPlus/JMEDPlus/JST7580(JDreamIII), Scopus

**C. DOCUMENTS CONSIDERED TO BE RELEVANT**

| Category* | Citation of document, with indication, where appropriate, of the relevant passages | Relevant to claim No. |
|---|---|---|
| X | WO 2007/145333 A1 (Tokyo Medical and Dental University), 21 December 2007 (21.12.2007), paragraphs [0007] to [0008], [0011], [0027] to [0034], [0059] to [0060] & US 2010/0062473 A1 paragraphs [0014] to [0015], [0018], [0057] to [0070], [0147] to [0172] & EP 2042867 A1 | 1-4 |
| A | KIDD, Parris, Th1/Th2 balance: the hypothesis, its limitations, and implications for health and disease, Alternative Medicine Review, 2003, 8(3), 223-246 | 1-4 |

☒ Further documents are listed in the continuation of Box C.     ☐ See patent family annex.

| * | Special categories of cited documents: |
|---|---|
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 31 October 2016 (31.10.16) | 08 November 2016 (08.11.16) |

| Name and mailing address of the ISA/ Japan Patent Office 3-4-3,Kasumigaseki,Chiyoda-ku, Tokyo 100-8915,Japan | Authorized officer Telephone No. |
|---|---|

Form PCT/ISA/210 (second sheet) (January 2015)

**INTERNATIONAL SEARCH REPORT**

| International application No. |
| --- |
| PCT/JP2016/078940 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 00/58728 A1  (Akitane UNO),<br>05 October 2000 (05.10.2000),<br>entire text; all drawings<br>& AU 3326000 A | 1-4 |
| A | WO 96/03523 A1  (NATIONAL INSTITUTES OF HEALTH),<br>08 February 1996 (08.02.1996),<br>entire text; all drawings<br>& EP 772692 A1 | 1-4 |
| A | WO 2014/005909 A1  (SOTIO A.S.),<br>09 January 2014 (09.01.2014),<br>entire text; all drawings<br>& US 2015/0337384 A1 | 1-4 |
| A | WO 2014/170497 A2  (EPIONTIS GMBH),<br>23 October 2014 (23.10.2014),<br>entire text; all drawings<br>& US 2016/0024578 A1 | 1-4 |
| A | WO 03/006956 A2  (NATIONAL HEALTH LABORATORY SERVICE),<br>23 January 2003 (23.01.2003),<br>entire text; all drawings<br>& EP 1405073 A2 | 1-4 |
| A | WO 2013/164823 A1  (MEDIAL RESEARCH LTD.),<br>07 November 2013 (07.11.2013),<br>entire text; all drawings<br>& US 2014/0235956 A1 | 1-4 |
| A | WO 2012/097374 A1  (CB BIOTECHNOLOGIES, INC.),<br>19 July 2012 (19.07.2012),<br>entire text; all drawings<br>& EP 2663864 A1 | 1-4 |
| A | JP 2-193069 A  (Japan Immuno Research Laboratories Co., Ltd.),<br>30 July 1990 (30.07.1990),<br>entire text; all drawings<br>& US 5725768 A | 1-4 |

Form PCT/ISA/210 (continuation of second sheet) (January 2015)

**EP 3 349 006 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014020930 A
- WO 0058728 A
- JP 2015141469 A
- JP 2015218530 A